# EUROPEAN PATENT SPECIFICATION

(11) **EP 3 618 813 B1**
(45) Date of publication and mention of the grant of the patent: **06.07.2022**
(21) Application number: 18725989.0
(22) Date of filing: 01.05.2018
(51) Int. Cl.: A61K 9/20, A61K 31/138, A61K 31/155, A61K 31/4015

(54) **IMPROVED EXTENDED RELEASE HIGHLY LOADED DRUG COMPOSITIONS**
VERBESSERTE WIRKSTOFFZUBEREITUNG MIT VERLANGSAMTER FREISETZUNG UND HOHER BELADUNG
COMPOSITION AMELIOREE FORTEMENT CHARGEE AVEC UNE LIBERATION PROLONGEE

(30) Priority: 02.05.2017 US 201762500078 P
(43) Date of publication of application: 11.03.2020
(73) Proprietor: Lubrizol Advanced Materials, Inc., Cleveland, OH 44141-3247 (US)
(72) Inventor: MIINEA, Liliana A., Cleveland, Ohio 44141-3247 (US); DRAGANOIU, Elena S., Vaughan, Ontario L4L 8K8 (CA); CHIKHALIKAR, Kedar V., Mumbai 400 079 (IN); CHADAWAR, Vikrant V., Mumbai 400 079 (IN)
(74) Representative: dompatent von Kreisler Selting Werner - Partnerschaft von Patent- und Rechtsanwälten mbB
(86) International application number: PCT/US2018/030380
(87) International publication number: WO 2018/204317

(56) References cited:
- EP-A1- 1 843 761
- EP-B1- 1 843 761
- WO-A1-2004/069234
- WO-A2-2006/082523
- WO-A2-2007/002516
- GB-A- 831 897
- US-A- 4 664 915

## Description

### FIELD OF THE INVENTION

There is provided herein improved drug compositions comprising a drug component, an extended release component and a buffering component that provide increased stability, higher drug loading potential, and suitable drug dissolution properties, and methods of making the same.

### BACKGROUND

Pharmaceutically active compounds, i.e. drugs, may be formulated for administration by numerous routes. Typically, the appropriate route will depend on the disease being treated, the chemical and physical properties of the pharmaceutically active substance as well as the subjects to be treated. Suitable pharmaceutical formulations include those designed for oral, rectal, nasal, topical, vaginal or parenteral administration or in a form suitable for administration by inhalation or insufflation.

Tablets, and especially tablets for oral administration, offer several advantages to both the manufacturer and to the patient. Tablets may be manufactured economically and are conveniently shipped, stored and dispensed. The patient can take advantage of a dosage form, which can be produced with an accurate dosage and has ease of administration and portability.

There is a need for improved tablet compositions that effectively delivers a drug to a patient while having a higher concentration of the pharmaceutically active substance. The higher concentration allows for smaller tablet sizes that still provide the same dosage. This makes the pills easier to swallow, which improves patient compliance and better management of dose regimen in the treatment of chronic diseases. However, increasing the concentration of the pharmaceutically active substance often causes the composition to have reduced stability and/or poor dissolution properties. Thus, there is a need for improved tablet compositions that effectively delivers a drug to a patient while having a higher concentration of the pharmaceutically active substance, where the tablet composition still has acceptable stability and dissolution properties.

WO 2007/002516 relates to drug compositions and dosage forms comprising said drug composition for the treatment of certain neural disorders / diseases, such as Parkinson's disease and other motor disorders with said pharmaceutical composition comprising (1) a first immediate-release (IR) portion formulated to provide a therapeutically effective concentration of a drug in the patient within 2 hours of administration and (2) a second portion formulated to release the drug at a substantially zero-order release rate over a sustained treatment period. WO 2006/082523 relates to an extended release dosage form of a water-soluble antidiabetic drug or its pharmaceutically acceptable salts and methods for preparing said extended release dosage form with said extended release dosage form comprising a hydrophilic polymer, an inorganic silicate and one or more pharmaceutically acceptable excipients.

### SUMMARY

The disclosed technology provides improved drug compositions that provide increased stability, higher drug loading potential, and suitable drug dissolution properties, and methods of making the same. The present invention and its preferred embodiments are apparent from the appendant set of claims.

The disclosed technology provides a composition that includes: (a) a drug component that includes a therapeutically effective amount of a drug; (b) an extended release component that includes a cross-linked polyacrylic acid polymer that is a carbomer homopolymer, carbomer copolymer, carbomer interpolymer, polycarbophil or a mixture thereof; and (c) a buffering component that includes magnesium hydroxide, magnesium oxide or any combination thereof: where the drug component makes up at least 50% by weight of the composition. The invention is also directed to a method of making a modified release tablet, wherein said method comprises the steps of: I. combining: (a) a drug component comprising a therapeutically effective amount of a drug; (b) an extended release component comprising a cross-linked polyacrylic acid polymer that is a carbomer homopolymer, carbomer copolymer, carbomer interpolymer, polycarbophil or a mixture thereof; and (c) a buffering component comprising magnesium hydroxide, magnesium oxide, or any combination thereof; wherein the drug component makes up at least 50% by weight of the composition; and II. using direct compression, granulation, or a combination thereof, to form the composition into a tablet. Further, the invention is directed to that composition for use as a medicament, wherein said composition has been formed into a tablet using direct compression, granulation, or a combination thereof.

The disclosed technology further provides for the described compositions where the drug of the drug component is subject to hydrolytic degradation.

The disclosed technology further provides for the described compositions where the drug of said drug component includes (i) an ester functional group, (ii) a lactone functional group, (iii) an amide or amide related functional group, (iv) a reactive nitrogen functional group, or (v) any combination thereof.

The disclosed technology further provides for the described compositions where the drug of said drug component includes: (i) Methylphenidate, Aspirin, Procaine, Benzocaine, Physostigmine, Tetracaine, N-methyl-dopa, Scopolamine, Meperidine, Steroid esters such as hydrocortisone sodium succinate, and methylprednisolone sodium succinate, succinylcholine chloride, chlorphenesin carbamate, carmethizole, cyclodisone, Estramustine, Carzelesin, hydrocortisone disodium phosphate, echothiophate Iodide, nitroglycerin, nicorandil, Phosphatidylcholine, phosphatidylethanolamine or any related compound; (ii) Lovastatin, Simvastatin, Daptomycin, Pilocarpine, Dalvastatin, Warfarin, and Camptothecin or any related compound; (iii) Acetamide, Chloramphenicol, Indomethacin, Lidocaine, Prazosin, Doxazosin, Dibucaine, acetaminophen, lincomycin, sulfacetamide, moricizin, Amoxicillin, Ampicillin, Latamoxef, benzylpenicillin, carbenicillin, phenethicillin, methicillin, cephems, such as cephalothin, cefadroxil, cephradine, and cefotaxime Cefepime, Cefaclor or other penicillins and cephalosporins any related compounds; peptides, polypeptides and proteins, amide related functional groups in levetiracetam, barbital, phenobarbital, amobarbital, metharbital, allantoin, Obidoxime, Doxorubicin, Tobramycin, or any related compounds; (iv) Benzodiazepines such as diazepam, oxazepam, nitrazepam, chlordiazepoxide, Triazolam, oxazolam, flutazolam, haloxazolam, cloxazolam, or any combination thereof; biguaninides such as metformin, phenformin, buformin, or any combination thereof; metoprolol, propranolol, bisoprolol, sotalol, atenolol, sulpyrine, furosemide, thiamine hydrochloride, diethylpropion, mitomycin C, zileuton, cifenline, Nitrofurantoin, rifampicin, chlorothiazide, hydrochlorothiazide, 5-azacytidine, cytarabine, or any related compounds; or (v) any combinations thereof.

The disclosed technology further provides for the described compositions where the drug or said drug component includes Metformin.

The disclosed technology further provides for the described compositions where the extended release component includes a cross-linked polyacrylic acid polymer that is a carbomer homopolymer, carbomer copolymer, carbomer interpolymer, polycarbophil or a mixture thereof. In some embodiments these extended release components can be referred to as carbomers, (i.e. crosslinked polyacrylic acid polymers) and hydrophobically modified derivatives thereof, sold under the registered trademark Carbopol^{®}, such as Carbopol 971P NF, Carbopol 71G NF, Carbopol 974P NF, Noveon AA-1 Polycarbophil, Carbopol^{®} 980NF polymer, Carbopol^{®} 940NF polymer, Carbopol 5984 EP, Carbopol 981NF, Carbopol^{®} Ultrez 10NF polymer, Carbopol^{®} Ultrez 21 polymer, Carbopol^{®} Ultrez 20 polymer, Carbopol^{®} ETD 2020NF polymer, Pemulen TR1NF, Pemulen TR2 NF, and the like commercially available from Lubrizol Advanced Materials, Inc.

According to the present invention, the buffering component includes magnesium hydroxide, magnesium oxide, or any combination thereof.

The disclosed technology further provides for the described compositions where the drug component makes up from 50 to 85 percent by weight of the composition. The disclosed technology further provides for the drug component making up from 50 to 85, from 55 to 85, from 65 to 85, to 70 to 85, from 75 to 85, or from 80 to 85 percent by weight of the drug composition, or even about 63, 67, or 80 percent by weight of the drug composition.

The disclosed technology further provides for the described compositions where: (a) the drug component makes up 50 to 85 percent by weight of the drug composition, or even from 50 to 85, 55 to 85, 60 to 85, 65 to 85, or 70 to 85 percent by weight; (b) the extended release component makes up 3 to 40 percent by weight of the drug composition, or from 3 to 40, 4 to 25, or 5 to 20 percent by weight; (c) the buffering component makes up 1 to 20 percent by weight of the drug composition, or from 1 to 20, 2 to 10, 3 to 7, or from 4 to 5 percent by weight; and further where said composition may optionally further include one or more additional additives.

The disclosed technology further provides for the described compositions where the composition has improved stability, as indicated by a lower level of impurities in the composition after exposure to forced degradation testing conditions; where the forced degradation testing conditions includes exposing the composition, in the form of a tablet, to 60 to 80 degrees C and 75% relative humidity for a period of 5 or 12 days; and where said lower level of impurities is measured by high pressure liquid chromatography (HPLC) analysis and compared to the level of impurities found when the same composition is tested without said buffering component.

The disclosed technology further provides for the described compositions where the composition is in a dosage form of tablets (mono, bi or multi-layered, coated or uncoated), capsules, granules, beads, or an aqueous dispersion.

The disclosed technology further provides for the described compositions where the composition has a pH level of 4 to 10, or even from 4 to 10, 5 to 10, 4 to 9, 5 to 9, 4 to 8, or even 5 to 8, where the pH level is measured by testing an aqueous dispersion of the tablet using a pH-meter; where the composition exhibits no more than 1 percent by weight impurities after forced degradation or even from 0 to 1, 0.01 to 1, or from 0 to 0.5, or from 0.01 to 0.5, or from 0 to 0.4, or from 0.01 to 0.4 percent by weight; where forced degradation comprises exposing the composition, in the form of a tablet, to 60 to 80 degrees C and 75% relative humidity for a period of 5 or 12 days; and where the level of impurities is measured by high pressure liquid chromatography (HPLC) analysis.

In some embodiments, the high-pressure liquid chromatography (HPLC) analysis described herein for Metformin API is completed using an Agilent 1260 Infinity Quaternary LC VL (Agilent Technologies). The mobile phase is a mixture of 90 volumes of buffer solution (pH 3.85) and 10 volumes of Acetonitrile. In some of these embodiments, the chromatographic conditions may be described as shown in the table below:

| | |
|---|---|
| HPLC | Agilent 1260 Infinity Quaternary LC VL (Agilent Technologies) |
| Column | Inertsil ODS 3 C₁₈ (25 cm x 4.6 mm) 5 µm or equivalent |
| Wavelength | 218 nm |
| Detector | Diode Array Detector |
| Flow rate | 1.0 mL / minute |
| Injection volume | 20 µl |
| Retention Time of Metformin HCl | About 10.0 minutes |
| Run Time for test solution | 30.0 minutes |
| Run Time for standard solution | 15.0 minutes |
| Diluent | Water : Acetonitrile (975:25) |

The standard solution used may be prepared as follows: accurately weigh about 25mg of Metformin HCl API in 100mL volumetric flask, add 60 mL of diluent and sonicate till it get dissolve completely. Cool and make up to the volume with diluent. (Standard Stock Solution 250ppm). Next, dilute 2mL of standard stock solution to 20mL with diluent. (Standard Solution (a) 25ppm). Dilute 1mL of standard solution (a) to 100mL with diluent (Standard solution 0.25ppm).

The test solutions may be prepared as follows: weigh accurately a quantity of powdered tablets about 625 mg equivalent to 500 mg of Metformin HCl, transfer in 100 mL of volumetric flask, add 60 mL of diluent and sonicate 30 minutes with intermittent swirling, cool to room temperature, make up the volume with diluent and centrifuge for 15 min @ 3500RPM. Dilute 5mL of supernatant to 100mL with diluent, filter the solution with 0.45 µm PVDF Millipore membrane syringe filter (250ppm).

In some embodiments, the high-pressure liquid chromatography (HPLC) analysis described herein for Levetiracetam API is completed using an Agilent 1260 Infinity Quaternary LC VL (Agilent Technologies). 1.4g of anhydrous di basic sodium phosphate in 1 liter of water. Adjusted to pH 3.5 with Orth phosphoric acid. Filter buffer with 0.45um Nylon membrane filter paper. A mixture of 95 volumes of buffer solution and 5 volumes of Acetonitrile. To each 1 Liter of the mixture, add 1 g of sodium 1-hexanesulfonate monohydrate. In some of these embodiments, the chromatographic conditions may be described as shown in the table below:

| | |
|---|---|
| HPLC | Agilent 1260 Infinity Quaternary LC VL (Agilent Technologies) |
| Column | Inertsil ODS 3V C₁₈ (25 cm x 4.6 mm) 5µm or equivalent |
| Wavelength | 205 nm |
| Detector | Diode Array Detector |
| Flow rate | 2.0 mL / minute |
| Injection volume | 20 µl |
| Retention Time of Levetiracetam | About 10.0 minutes |
| Run Time for test solution | 60.0 minutes |
| Run Time for standard solution | 20.0 minutes |
| Diluent | Solution A: Acetonitrile (95:5) |
| | Solution A : Dilute 2 mL of phosphoric acid with water) |

The standard solution used may be prepared as follows: Accurately weigh about 250 mg of Levetiracetam API in 100mL volumetric flask, add 70 mL of Water and sonicate till it get dissolve completely. Cool and make up to the volume with water. (Standard Stock Solution 2500 ppm). Next, Dilute 5mL of standard solution a to 50mL with water (Standard Solution (a) 250 ppm), further Dilute 5mL of standard solution (a) to 100mL with water (Standard Solution 12.5 ppm).

The test solutions may be prepared as follows: Weigh accurately a quantity of sample equivalent to 500 mg of Levetiracetam, transfer in 100 mL of volumetric flask, add 30 mL of acetonitrile and sonicate with intermittent swirling for 10 min and shake for 10 min, to that add 30mL of Water, and shake for 15 min using a mechanical shaker. Cool to room temperature and add 25 mL of acetonitrile to volumetric flask and make up the volume with water and centrifuge for 15 min @ 3500RPM. filter the solution with 0.45 µm PVDF Millipore membrane syringe filter by discarding first 5mL of filtrate. Dilute 5mL of filtrate to 10mL with water (2500ppm).

The tablet pH may be measured by crushing 1 tablet of the described drug compositions and adding 70 ml of deionized water. The dispersion is sonicated for 15 minutes followed by volume make up to 100 ml with deionized water. The pH of the resultant dispersion is measured at room temperature using a pH meter make: Lab India Model: PICO.

The disclosed technology further provides for the described compositions where in the composition is in the form of a tablet, which may be monolayer or multilayered; and where the tablet contains a dosage of said drug in the amount of 500 to 1000 mg.

The disclosed technology further provides for a method of making the described compositions and modified release tablets made of the described compositions, wherein said method includes the steps of: (I) combining: (a) a drug component that includes a therapeutically effective amount of a drug; (b) an extended release component that includes a cross-linked polyacrylic acid polymer that is a carbomer homopolymer, carbomer copolymer, carbomer interpolymer, polycarbophil or a mixture thereof; (c) a buffering component comprising magnesium hydroxide, magnesium oxide, or any combinations thereof; where the drug component makes up at least 50 % by weight of the composition; and (II) using direct compression, granulation, or a combination thereof, to form the composition into a tablet.

The disclosed technology further provides the above-described composition for use as a medicament, wherein said composition has been formed into a tablet using direct compression, granulation, or a combination thereof.

### DETAILED DESCRIPTION

Various preferred features and embodiments will be described below

The disclosed technology provides improved drug compositions that provide increased stability, higher drug loading potential, and suitable drug dissolution properties, and methods of making the same.

### The Drug Compositions

The drug compositions disclosed herein include: (a) a drug component that includes at least one therapeutically effective amount of a drug; (b) an extended release component that includes at least one cross-linked polyacrylic acid polymer that is a carbomer homopolymer, carbomer copolymer, carbomer interpolymer, polycarbophil or a mixture thereof; and (c) a buffering component comprising magnesium hydroxide, magnesium oxide, or any combination thereof. The compositions are also formulated such that the drug component makes up at least 50% by weight of the composition. In other embodiments the drug, which may also be referred to as the pharmaceutically active compound, in the drug component makes up at least 50% by weight of the composition. In still other embodiments the drug component contains at least two drugs and the combined amount of all the drugs present in the composition makes up at least 50% by weight of the composition.

In still other embodiments, the drug component (or the drug or drugs themselves) make up from 50% to 85% by weight of the composition.

In some embodiments, component (a), the drug component, may make up 50% to 85% by weight of the drug composition. Component (b), the extended release component, may make up 3% to 40% by weight of the drug composition. Component (c), the buffering component, may make up 1% to 20% by weight of the drug composition. In any of these embodiments, the composition may further include one or more additional additives.

The composition may be used in various forms. However, in some embodiments, the composition is in the form of a tablet. In still further embodiments the composition is in the form of a tablet designed for oral administration.

The purpose of the disclosed technology is to provide improved drug compositions, where the drug compositions have an acceptable balance of one or more properties, including the stability of the composition, the active component loading of the composition, and the dissolution properties of the composition. In some embodiments, the disclosed technology provides drug compositions with improved active component loading, that is, higher concentrations of the active pharmaceutical agent, or drug. In some embodiments, the disclosed technology provides drug compositions with improved stability. In some embodiments, the disclosed technology provides drug compositions with improved dissolution properties. In some embodiments, the disclosed technology provides drug compositions with an improvement in one or more of the areas described above and no harm to the other areas. For example, in some embodiments the disclosed technology provides drug compositions with improved active component loading with no harm to the stability of the composition and no harm to the dissolution properties of the composition. In still further embodiments the disclosed composition provides a commercially useful balance of all three of these properties. It is very difficult to balance these factors in a way that produces useful and effective drug compositions. High active component loading often has a negative impact on the stability of the composition and/or the dissolution properties of the composition, and vice versa. Developing drug compositions that provide a better balance of all of these properties would result in very useful compositions, and that is the goal of the disclosed technology.

With regards to the stability of the drug compositions, in some embodiments, this is evaluated by measuring the level of impurities that form in the drug composition over time, with lower impurity levels indicating better stability. In addition, in some embodiments, the drug compositions are exposed to storage conditions designed to accelerate and/or force the degradation of the drug composition, which is referred to herein as forced degradation testing conditions.

In some embodiments, the forced degradation testing conditions include: exposing the composition, in the form of a tablet, to 60 to 80 degrees C and 75% relative humidity for a period of 5 or 12 days. The level of impurities is then measured by high-pressure liquid chromatography (HPLC) analysis and compared to the level of impurities found in other compositions, including compositions that are identical except for the absence of one or more components, such as the buffering component. The lower level of impurities, as measured by HPLC, the better stability of the composition.

In some embodiments, the disclosed technology includes drug compositions that have a specific pH level. The pH level can be measured by making an aqueous dispersion of the tablet and then using a pH-meter to measure the pH of the resulting solution.

In some embodiments, the disclosed technology includes drug compositions that have: (i) a pH level of 4 to 10, or from 5 to 10, 4 to 9, 5 to 9, 4 to 8, or even 5 to 8; (ii) an impurity level, after the composition has been exposed to forced degradation testing conditions (as described above), of no more than 1% by weight impurities, or even from 0% or 0.1% percent by weight impurities up to 1%, 0.5%, 0.4% by weight impurities, as measured by HPLC (as described above).

The disclosed technology also provides for any of the drug compositions described herein where the composition is in the form of a tablet. In some embodiments, the tablet contains a dosage of the drug or drugs in the amount of 500 to 1000 mg.

The drug compositions disclosed herein may be used as controlled release drug delivery system and can be formed into any suitable dosage form. Such forms include, but are not limited to, mono-layered or multi-layered tablets, pills, capsules, gel-tabs, granules, beads, or an aqueous dispersion of one or more thereof. Depending upon the desired dosage form for the drug compositions of the present invention various manufacturing methods can be used. Such methods include, but are not limited to, direct compression, wet granulation, roller compaction, hot-melt granulation, fluid bed granulation or the like. In some embodiments, the drug compositions of the present invention are in the form of tablets.

The disclosed technology provides for the described drug compositions where: (a) the drug component makes up 50 to 85 percent by weight of the drug composition, or even from 50 to 85, 55 to 85, 60 to 85, 65 to 85, or 70 to 85 percent by weight, or even about 63, 67, or even 80 percent by weight of the drug composition; (b) the extended release component makes up 3 to 40 percent by weight of the drug composition, or from 3 to 40, 4 to 25, or 5 to 20 percent by weight; (c) the buffering component makes up 1 to 20 percent by weight of the drug composition, or from 1 to 20, 2 to 10, 3 to 7, or from 4 to 5 percent by weight; and further where said composition may optionally further include one or more additional additives.

### The drug component

Component (a), the drug component, includes a therapeutically effective amount of a drug. In some embodiments, the drug component is made up of a drug and does not include any other materials or components. In other embodiments, the drug component is made up of two or more drugs (and sometimes just two drugs) and does not include any other materials or components. In other embodiments, the drug component is made up of three or more drugs (and sometime just three drugs) and does not include any other materials or components.

The drugs useful in the compositions described herein are not overly limited. Any therapeutically useful compound that can administered to a patient may be suitable for use in the compositions described herein.

In some embodiments described herein, at least one drug present in the drug component is subject to hydrolytic degradation. In other embodiments, all of the drugs present in the drug component are subject to hydrolytic degradation. In still other embodiments, at least one drug present in the drug component is subject to hydrolytic degradation and at least one drug present in drug component is not subject to hydrolytic degradation.

In some embodiments, the drug or drugs present in the drug component include (i) an ester functional group, (ii) a lactone functional group, (iii) an amide or amide related functional group, (iv) a reactive nitrogen functional group, or (v) any combination thereof.

In some embodiments, the drug of said drug component includes one or more of the following: (i) Methylphenidate, Aspirin, Procaine, Benzocaine, Physostigmine, Tetracaine, N-methyl-dopa, Scopolamine, Meperidine, Steroid esters such as hydrocortisone sodium succinate, and methylprednisolone sodium succinate, succinylcholine chloride, chlorphenesin carbamate, carmethizole, cyclodisone, Estramustine, Carzelesin, hydrocortisone disodium phosphate, echothiophate Iodide, nitroglycerin, nicorandil, Phosphatidylcholine, phosphatidylethanolamine or any related compound;

In some embodiments, the drug of said drug component includes one or more of the following: (ii) Lovastatin, Simvastatin, Daptomycin, Pilocarpine, Dalvastatin, Warfarin,and camptothecin or any related compound.

In some embodiments, the drug of said drug component includes one or more of the following: (iii) Acetamide, Chloramphenicol, Indomethacin, Lidocaine, Prazosin, Doxazosin, Dibucaine, acetaminophen, lincomycin, sulfacetamide, moricizin, Amoxicillin, Ampicillin, Latamoxef, benzylpenicillin, carbenicillin, phenethicillin, methicillin, cephems, such as cephalothin, cefadroxil, cephradine, and cefotaxime Cefepime, Cefaclor or other penicillins and cephalospoins any combination thereof; peptides, polypeptides and proteins, amide related functional groups in levetiracetam, barbital, phenobarbital, amobarbital, metharbital, allantoin, Obidoxime, Doxorubicin, Tobramycin, or any other related compound.

In some embodiments, the drug of said drug component includes one or more of the following: (iv) Benzodiazepines such as diazepam, oxazepam, nitrazepam, chlordiazepoxide, Triazolam, oxazolam, flutazolam, haloxazolam, cloxazolam, or any combination thereof; biguaninides such as metformin, phenformin, buformin, or any combination thereof; metoprolol, propranolol, bisoprolol, sotalol, atenolol, sulpyrine, furosemide, thiamine hydrochloride,diethylpropion, mitomycin C, zileuton, cifenline, Nitrofurantoin, rifampicin, chlorothiazide, hydrochlorothiazide, 5-azacytidine, cytarabine, or any other related compound.

Any combination of the drugs listed above may be used in the present invention. In some embodiments, the drug component includes Metformin. In still further embodiments, the only drug in the drug component is Metformin. In still further embodiments, the drug component is solely made up of Metformin.

In other embodiments, the drug component includes Levetiracetam. In still further embodiments, the only drug in the drug component is Levetiracetam. In still further embodiments, the drug component is solely made up of Levetiracetam.

In still other embodiments, the drug component includes Metoprolol. In still further embodiments, the only drug in the drug component is Metoprolol. In still further embodiments, the drug component is solely made up of Metoprolol.

### The extended release component

The drug compositions disclosed herein include an extended release component. The extended release component includes a cross-linked polyacrylic acid polymer that is a carbomer homopolymer, carbomer copolymer, carbomer interpolymer, polycarbophil or a mixture thereof.

The extended release component may include a cross-linked polyacrylic acid selected from one or more carbomers, one or more polycarbophils, one or more copolymers of acrylic acid and alkyl acrylates, or combinations of two or more thereof.

As used herein, the term polyacrylic acid or acrylic acid polymers is used to encompass a variety of polymers having high percentages of polymerizable monomers therein with pendant carboxylic acid groups or anhydrides of polycarboxylic acid. These compounds are described in more detail in U.S. Pat. Nos. 2,798,053; 3,915,921; 4,267,103; 5,288,814; and 5,349,030. The term polyacrylic acid is also used to include various homopolymers, copolymers, and interpolymers, wherein at least 50 or 75 mole percent of the repeating units have pendant carboxylic acid groups or anhydrides of dicarboxylic acid groups. While acrylic acid is the most common primary monomer used to form polyacrylic acid the term is not limited thereto but includes generally all alpha-beta-unsaturated monomers with carboxylic pendant groups or anhydrides of dicarboxylic acids as described in U.S. Pat. No. 5,349,030.

Suitable cross-linked polyacrylic acids include, but are not limited to polycarbophils, carbomers, Carbopol^{®} polymers, Carbopol homopolymers, Carbopol copolymers, Carbopol interpolymers copolymers of acrylic acid and alkyl acrylates, or combinations of two or more thereof. An approved polyacrylic acid for pharmaceutical applications, described in a carbomer monograph in the U.S.P. Pharmacopeia 30 NF 25, is a polyacrylic acid crosslinked with polyalkenyl ethers.

In some embodiments, the extended release component includes Carbopol 971P NF, Carbopol 71G NF, Carbopol 974P NF, or any combination thereof. These Carbomers are commercially available from Lubrizol Advanced Materials, Inc., Cleveland Ohio.

### The buffering component

The drug compositions disclosed herein include a buffering component. The buffering component **comprises** magnesium **hydroxide, magnesium oxide, or any combination thereof.** While other buffering agents may also be present in some embodiments, the disclosed technology requires the presence of magnesium **hydroxide, magnesium oxide, or any combination thereof.** In some embodiments, magnesium **hydroxide, magnesium oxide, or any combination thereof** is the only buffering agent present in the composition.

**According to the invention, the**
buffering component comprises magnesium hydroxide, magnesium oxide, or any combination thereof.

While not wishing to be bound by theory, it has been found that magnesium containing buffering agents, including the agents provided above, provide drug compositions with the benefits described herein, over and above the properties found in drug compositions that use other buffering agents and/or differ from the drug compositions described herein in some other way.

### Additional components

The drug compositions disclosed herein may include, in addition to the components described above, one or more pharmaceutically acceptable additives.

Useful pharmaceutically acceptable additives include diluents, binders, lubricants, glidants, coatings, preservatives, stabilizers, surfactants, colorants, disintegrants, plasticizers, modified release agents, controlled release agents, and the like.

More specific examples of useful additives include: hypromellose rate controlling polymers such as HPMC K100M which is commercially available from Colorcon; sodium carboxymethylcellulose such as Blanose^{™} 7HFPH which is commercially available from Ashland; microcrystalline cellulose such as Avicel pH 101 which is commercially available from FMC BioPolymer; hygroscopic, amorphous polymers such as PVP K-90 which is commercially available from Ashland; hydrophilic fumed silicas such as Aerosil^{®} 200 which is commercially available from Evonik Industries; and any combination thereof.

In some embodiments, the drug compositions disclosed herein include at least one enteric polymer.

Suitable enteric polymers include, but are not limited to, polyacrylate copolymers such as methacrylic acid copolymer, USP/NF, Types A, B or C (which are available from Evonik Industries AG under the brand name Eudragit^{®}); cellulose derivatives (e.g., cellulose acetate phthalate, cellulose acetate trimellitate, hydroxypropyl methylcellulose phthalate, or hydroxypropyl methylcellulose acetate succinates); polyvinyl acetate phthalate; shellac; or suitable combinations of two or more thereof.

In another embodiment, useful enteric polymers include, but are not limited to, pharmaceutically-acceptable forms of cellulose, vinyl, and acrylic polymer derivatives. These polymers exhibit resistance to gastric fluids yet are readily soluble or permeable in intestinal fluid. Enteric polymeric materials are primarily weak acids containing acidic functional groups, which are capable of ionization at elevated pH (above a pH of about 5). In the low pH of the stomach, enteric polymers remain unionized, and therefore, insoluble. As the pH increases in the intestinal tract, the functional groups present in enteric polymers ionize, and the polymer becomes soluble in the intestinal fluids.

### Methods

The disclosed technology includes methods of making any of the drug compositions described herein. Such methods include the steps of: Step (I) combining: (a) a drug component that includes a therapeutically effective amount of a drug; (b) an extended release component that includes a cross-linked polyacrylic acid polymer that is a carbomer homopolymer, carbomer copolymer, carbomer interpolymer, polycarbophil or a mixture thereof; and (c) a buffering component **comprising magnesium hydroxide, magnesium oxide, or any combination thereof.** As noted above, the drug compositions described herein are formulated such that the drug component makes up at least 50% by weight of the composition.

In some embodiments, the compositions are in a form designed for oral, rectal, nasal, topical, vaginal or parenteral administration or is in a form suitable for administration by inhalation or insufflation. In some embodiments, the drug compositions are in the form of a tablet. The disclosed technology includes methods of making the compositions into such forms, including tablets, made from any of the drug compositions described herein. Such methods include the steps of: Step (I) combining: (a) a drug component that includes a therapeutically effective amount of a drug; (b) an extended release component that includes a cross-linked polyacrylic acid polymer that is a carbomer homopolymer, carbomer copolymer, carbomer interpolymer, polycarbophil or a mixture thereof; (c) a buffering component comprising magnesium hydroxide, magnesium oxide, or any combination thereof; and Step (II) using direct compression, granulation, or a combination thereof, to form the composition into a tablet. In some embodiments, the method uses direct compression. As noted above, the drug compositions described herein are formulated such that the drug component makes up at least 50% by weight of the composition.

The disclosed technology also includes **the above-described composition for use as a medicament, wherein said composition has been formed into a tablet using direct compression, granulation, or a combination thereof.**

The amount of each chemical component described is presented exclusive of any solvent which may be customarily present in the commercial material, that is, on an active chemical basis, unless otherwise indicated. However, unless otherwise indicated, each chemical or composition referred to herein should be interpreted as being a commercial grade material which may contain the isomers, by-products, derivatives, and other such materials which are normally understood to be present in the commercial grade.

It is known that some of the materials described above may interact in the final formulation, so that the components of the final formulation may be different from those that are initially added. For instance, metal ions can migrate to other acidic or anionic sites of other molecules. The products formed thereby, including the products formed upon employing the composition of the technology described herein in its intended use, may not be susceptible of easy description.

The technology described herein encompasses the composition prepared by admixing the components described above.

### EXAMPLES

The technology described herein may be better understood with reference to the following examples.

### Materials

Various drug compositions were prepared to demonstrate the benefits of the disclosed technology.

The process used to prepare each of the examples is as follows: Metformin HCl, HPMC and buffers are screened through **40 µm** (40 mesh), blended and granulated with 2% w/w aqueous dispersion of Carbopol 971P NF polymer. The granules are dried at 60⁰C, sized through **841 µm** (20 mesh) sieve, blended with extragranular components (Carbopol 971P NF and/or Carbopol 71G NF polymers, buffers, colloidal silicon dioxide and magnesium stearate) and the blend is compressed into tablets.

A group of **comparative** examples is made using magnesium carbonate as the buffering agent. The formulations of these examples are summarized below, where all amounts are in percent by weight.

**Table 1: Magnesium Carbonate Examples (Comparative)**

| | EX 1 | EX 2 | EX 3 | EX 4 | EX 5 |
|---|---|---|---|---|---|
| Metformin (Metformin HCL) | 80 | 80 | 77.44 | 80 | 80 |
| HPMC K100M | 9.24 | 4.8 | 7.54 | 9.24 | 4.8 |
| Magnesium Carbonate | 4.2 | 4.8 | 5.02 | 4.2 | 4.8 |
| Carbopol^{®} 971P NF Polymer | 5.28 | 4.32 | 4.18 | 2.8 | 4.32 |
| Carbopol^{®} 71G NF Polymer | 0 | 4.8 | 4.62 | 2.48 | 4.8 |
| Aerosil^{™} 200 Fumed Silica | 0.72 | 0.72 | 0.69 | 0.72 | 0.72 |
| Magnesium Stearate | 0.56 | 0.56 | 0.54 | 0.56 | 0.56 |

Another group of examples is made using magnesium hydroxide as the buffering agent. The formulations of these examples are summarized below, where all amounts are in percent by weight.

**Table 2a: Magnesium Hydroxide Examples**

| | EX 6 | EX 7 | EX 8 | EX 9 | EX 10 |
|---|---|---|---|---|---|
| Metformin (Metformin HCL) | 77.44 | 69.43 | 80.54 | 77.44 | 80.54 |
| HPMC K100M | 4.62 | 10.34 | 1.6 | 7.54 | 4.8 |
| Blanose^{™} 7HFPH Sodium CMC | 0 | 3.2 | 3.2 | 0 | 0 |
| Magnesium Hydroxide | 4.1 | 6.9 | 4.26 | 5.02 | 4.26 |
| Carbopol^{®} 971P NF Polymer | 4.17 | 4.32 | 4.32 | 4.17 | 4.32 |
| Carbopol^{®} 71G NF Polymer | 4.62 | 4.62 | 4.8 | 4.62 | 4.8 |
| Sodium Chloride | 3.85 | 0 | 0 | 0 | 0 |
| Magnesium Oxide | 0 | 0 | 0 | 0 | 0 |
| Aerosi^{™}1 200 Fumed Silica | 0.69 | 0.69 | 0.72 | 0.69 | 0.72 |
| Magnesium Stearate | 0.54 | 0.52 | 0.56 | 0.54 | 0.56 |

**Table 2b: Magnesium Hydroxide Examples**

| | EX 11 | EX 12 | EX 13 | EX 14 | EX 15 |
|---|---|---|---|---|---|
| Metformin (Metformin HCL) | 77.44 | 77.44 | 77.44 | 77.44 | 80 |
| HPMC K100M | 8.47 | 0 | 7.54 | 7.54 | 4.8 |
| Blanose^{™} 7HFPH Sodium CMC | 0 | 8.46 | 0 | 0 | 0 |
| Magnesium Hydroxide | 4.1 | 4.1 | 3.28 | 2.51 | 4.8 |
| Carbopol^{®} 971P NF Polymer | 4.17 | 4.17 | 4.16 | 4.17 | 4.32 |
| Carbopol^{®} 71G NF Polymer | 4.62 | 4.62 | 4.62 | 4.62 | 4.8 |
| Sodium Chloride | 0 | 0 | 0 | 0 | 0 |
| Magnesium Oxide | 0 | 0 | 1.74 | 2.5 | 0 |
| Aerosil^{™} 200 Fumed Silica | 0.69 | 0.69 | 0.69 | 0.69 | 0.72 |
| Magnesium Stearate | 0.54 | 0.54 | 0.54 | 0.54 | 0.56 |

Another group of examples is made using magnesium oxide as the buffering agent. The formulations of these examples are summarized below, where all amounts are in percent by weight.

**Table 3: Magnesium Oxide Examples**

| | EX 16 | EX 17 | EX 18 | EX 19 | EX 20 |
|---|---|---|---|---|---|
| Metformin (Metformin HCL) | 80 | 76.92 | 77.44 | 77.44 | 78.74 |
| HPMC K100M | 4.8 | 4.62 | 7.54 | 7.54 | 4.72 |
| Magnesium Oxide | 0 | 0 | 3.28 | 2.51 | 0 |
| Avicel^{™} pH 101 MCC | 0 | 0.96 | 0 | 0 | 0 |
| PVP K-90 Poly(vinylpyrrolidone) | 0 | 1.54 | 0 | 0 | 0 |
| Carbopol^{®} 971P NF Polymer | 4.32 | 7.31 | 4.16 | 4.17 | 4.26 |
| Carbopol^{®} 71G NF Polymer | 4.8 | 3.6 | 4.62 | 4.62 | 6.3 |
| Magnesium Oxide | 4.8 | 3.85 | 1.74 | 2.5 | 4.72 |
| Aerosil^{™} 200 Fumed Silica | 0.72 | 0.72 | 0.69 | 0.69 | 0.71 |
| Magnesium Stearate | 0.56 | 0.48 | 0.54 | 0.54 | 0.55 |

Another group of **comparative** examples is made using buffering agents that do not contain magnesium. The formulations of these examples are summarized below, where all amounts are in percent by weight.

**Table 4a: Examples with Other Buffering Agents (Comparative)**

| | EX 21 | EX 22 | EX 23 | EX 24 | EX 25 |
|---|---|---|---|---|---|
| Metformin (Metformin HCL) | 83.33 | 80 | 80 | 80 | 80 |
| HPMC K100M | 5 | 4.8 | 4.8 | 4.8 | 4.8 |
| Avicel^{™} pH 101 MCC | 1.04 | 1 | 0 | 0 | 0 |
| PVP K-90 Poly(vinylpyrrolidone) | 1.67 | 1.6 | 0 | 0 | 0 |
| Carbopol^{®} 971P NF Polymer | 1.67 | 3.75 | 4.32 | 4.32 | 4.32 |
| Carbopol^{®} 71G NF Polymer | 1 | 2.8 | 4.8 | 4.8 | 4.8 |
| Sodium bicarbonate | 5 | 2.4 | 4.8 | 0 | 0 |
| Sodium hydrogen phosphate | 0 | 0 | 0 | 0 | 0 |
| Sodium acetate trihydrate | 0 | 0 | 0 | 0 | 0 |
| Trisodium phosphate | 0 | 0 | 0 | 0 | 0 |
| Calcium carbonate | 0 | 0 | 0 | 4.8 | 0 |
| Potassium bicarbonate | 0 | 0 | 0 | 0 | 4.8 |
| Aerosil^{™} 200 Fumed Silica | 0.77 | 0.75 | 0.72 | 0.72 | 0.72 |
| Magnesium Stearate | 0.52 | 0.5 | 0.56 | 0.56 | 0.56 |

**Table 4b: Examples with Other Buffering Agents (Comparative)**

| | EX 26 | EX 27 | EX 28 |
|---|---|---|---|
| Metformin (Metformin HCL) | 80.8 | 77.44 | 77.44 |
| HPMC K100M | 4.8 | 8.46 | 8.46 |
| Avicel^{™} pH 101 MCC | 0 | 0 | 0 |
| PVP K-90 Poly(vinylpyrrolidone) | 0 | 0 | 0 |
| Carbopol^{®} 971P NF Polymer | 4.32 | 4.17 | 4.17 |
| Carbopol^{®} 71G NF Polymer | 4.8 | 4.62 | 4.62 |
| Sodium bicarbonate | 0 | 0 | 0 |
| Sodium hydrogen phosphate | 4 | 0 | 0 |
| Sodium acetate trihydrate | 0 | 4.09 | 0 |
| Trisodium phosphate | 0 | 0 | 4.09 |
| Calcium carbonate | 0 | 0 | 0 |
| Potassium bicarbonate | 0 | 0 | 0 |
| Aerosil^{™} 200 Fumed Silica | 0.72 | 0.69 | 0.69 |
| Magnesium Stearate | 0.56 | 0.54 | 0.54 |

In all of the examples herein: HPMC K100M and HPMC K4M are a hypromellose rate controlling polymers commercially available from The Dow Chemical Company; Blanose^{™} 7HFPH is sodium carboxymethylcellulose commercially available from Ashland; Carbopol^{®} 971P NF and Carbopol^{®} 71G NF are cross-linked polyacrylic acid polymers commercially available from Lubrizol Advanced Materials, Inc.; Avicel pH 101 is a microcrystalline cellulose commercially available from FMC BioPolymer; PVP K-90 is a hygroscopic, amorphous polymer commercially available from Ashland; Aerosil^{®} 200 is a hydrophilic fumed silica commercially available from Evonik Industries; Neusilin^{™} UFL2 is an amorphous magnesium aluminometasilicate commercially available from Fuji Chemical Industries Co., Ltd.

This group of examples is subjected to forced degradation testing conditions, where each example is formed into a tablet and then stored at 60 to 80 degrees C and 75% relative humidity for a period of 5 or 12 days. At the end of the 5 days or 12 days each tablet is tested for its pH level and percent impurities. Each sample is exposed in an open petri dish inside a humidity chamber (Espec Corporation Japan, Model LHU -113) that is controlled at 80 Degrees C and 75 % RH condition for 5 days or 12 days. Addition mode in Table 5 below indicated the means of addition of the buffering agent during the preparation of the tablets of the examples.

The results of this testing are presented in the table below.

**Table 5: Results from Examples**

| EX. ID | BUFFER ID | WT% BUFFER | pH LEVEL | WT% IMPURITY | ADDITION MODE | PHYSICAL STABILITY¹ |
|---|---|---|---|---|---|---|
| EX 1^{∗} | Mg Carbonate | 4.2 | 6.3 | 0.1 | Intragranular | No |
| EX 2∗ | Mg Carbonate | 4.8 | 5.6 | 0.5 | Intragranular | No |
| EX 3∗ | Mg Carbonate | 5.0 | 4.9 | 0.7 | Intragranular | No |
| EX 4∗ | Mg Carbonate | 4.2 | 5.9 | 0.1 | Extragranular | No |
| EX 5∗ | Mg Carbonate | 4.8 | 5.6 | 0.6 | Extragranular | No |
| EX 6 | Mg Hydroxide | 4.1 | 6.2 | 0.0 | Intragranular | Yes |
| EX 7 | Mg Hydroxide | 6.9 | 5.6 | 0.1 | Intragranular | Yes |
| EX 8 | Mg Hydroxide | 4.3 | 6.5 | 0.1 | Intragranular | Yes |
| EX 9 | Mg Hydroxide | 5.0 | 6.5 | 0.2 | Intragranular | Yes |
| EX 10 | Mg Hydroxide | 4.3 | 6.7 | 0.2 | Intragranular | Yes |
| EX 11 | Mg Hydroxide | 4.1 | 6.2 | 0.2 | Intragranular | Yes |
| EX 12 | Mg Hydroxide | 4.1 | 6.0 | 0.2 | Intragranular | Yes |
| EX 13 | Mg Hydroxide | 3.3 | 10.0 | 0.3 | Intragranular | Yes |
| EX 14 | Mg Hydroxide | 2.5 | 8.6 | 0.3 | Intragranular | Yes |
| EX 15 | Mg Hydroxide | 4.8 | 6.5 | 0.1 | Extragranular | Yes |
| EX 16 | Mg Oxide | 4.8 | 6.4 | 0.1 | Extragranular | Yes |
| EX 17 | Mg Oxide | 3.9 | 6.1 | 0.3 | Extragranular | Yes |
| EX 18 | Mg Oxide | 1.7 | 10.0 | 0.3 | Extragranular | Yes |
| EX 19 | Mg Oxide | 2.5 | 8.6 | 0.3 | Extragranular | Yes |
| EX 20 | Mg Oxide | 4.7 | 6.1 | 0.3 | Extragranular | Yes |
| EX 21^{∗} | Na Bicarbonate | 6.1 | 5.0 | 0.2 | Extragranular | No |
| EX 22∗ | Na Bicarbonate | 2.4 | 6.0 | 0.4 | Extragranular | No |
| EX 23* | Na Bicarbonate | 4.8 | 5.6 | 0.7 | Extragranular | No |
| EX 24* | Ca Carbonate | 4.8 | 5.4 | 0.9 | Extragranular | No |
| EX 25* | K Bicarbonate | 4.8 | 5.4 | 0.8 | Extragranular | No |
| EX 26^{∗} | Na Hydrogen Phosphate | 4.0 | 6 | 0.90 | Extragranular | Yes |
| EX 27^{∗} | Na Acetate·3H₂O | 4.09 | 4.7 | 0.97 | Extragranular | Yes |
| EX 28∗ | Trisodium Phosphate | 4.09 | 5.6 | 0.99 | Extragranular | Yes |

| | | | | | | |
|---|---|---|---|---|---|---|
| **^{∗}Reference Example** ¹Physical stability was visually evaluated for evidence of outgassing (CO₂ evolution). Samples that evolved CO₂ failed the stability test. | | | | | | |

The results show that the compositions of the disclosed technology provide a better balance of properties (low impurities and good physical stability) important to drug compositions.

Also provided are additional examples using a different active agent, Levetiracetam. The formulations of these examples are summarized below, where all amounts are in percent by weight. The ingredients of Examples 29 and 30 were tableted while the ingredients of Examples 31, 32, and 33 were powder blends.

The tablets were prepared as follows. Levetiracetam, HPMC and magnesium hydroxide are screened through a 400 µm (40-mesh) sieve, blended and granulated with 2% w/w aqueous dispersion of Carbopol 971P NF polymer. The granules were dried at 60°C, sized through **841 µm** (20-mesh) sieve and blended with extragranular component: (Carbopol 971P NF and Carbopol 71G NF polymers, colloidal silicon dioxide and magnesium stearate). The resulting blend of Examples 29 and 30 was compressed into tablets.

**Table 6: Examples with Levetiracetam**

| | EX 2 9^{∗} | EX 30 | EX 31^{∗} | EX 32^{∗} | EX 33¹ |
|---|---|---|---|---|---|
| Levetiracetam | 77.44 | 77.44 | 100 | 50 | 33.4 |
| HPMC K100M | 12.56 | 8.46 | 0 | 0 | 0 |
| Magnesium Hydroxide | 0 | 4.1 | 0 | 0 | 33.3 |
| Carbopol^{®} 971P NF Polymer | 4.17 | 4.17 | 0 | 50 | 33.3 |
| Carbopol^{®} 71G NF Polymer | 4.62 | 4.62 | 0 | 0 | 0 |
| Aerosil^{™} 200 Fumed Silica | 0.69 | 0.69 | 0 | 0 | 0 |
| Magnesium Stearate | 0.54 | 0.54 | 0 | 0 | 0 |

| | | | | | |
|---|---|---|---|---|---|
| ¹Comparative ^{∗} **Reference Example** | | | | | |

These examples are prepared and tests as noted above except that they are exposed to the forced degradation conditions described previously for a period of 12 days. The results of these examples are provided in the table below.

**Table 7: Results from Examples**

| EXAMPLE ID | BUFFER ID | WT% BUFFER | WT% IMPURITY |
|---|---|---|---|
| EX 29 ^{∗} | Mg Hydroxide | 0 | 0.66 |
| EX 30 | Mg Hydroxide | 4.1 | 0.11 |
| EX 31 ∗ | Mg Hydroxide | 0 | 0.02 |
| EX 32 ∗ | Mg Hydroxide | 0 | 7.15 |
| EX 33¹ | Mg Hydroxide | 33.3 | 4.59 |

| | | | |
|---|---|---|---|
| ¹Comparative ^{∗} **Reference Example** | | | |

Blends of Levetiracetam (API) were prepared (at ambient room temperature and relative humidity) in the various blend ratios from the components set forth in Table 8. The blends were subjected to forced degradation testing where a sample of each blend was stored for 12 days at 60°C and 75% relative humidity (RH) (12 Day Exposure) and for 30 days at 40°C and 75% RH (30 Day Exposure). At the end of the storage periods, each sample was analyzed for Levetiracetam acid (a degradation product of Levetiracetam) and total impurities. At the onset of the forced degradation testing, an initial analysis of each sample was conducted to determine baseline amounts of Levetiracetam acid and total impurities present in each blend before the storage stability testing. The amount of Levetiracetam acid and total impurities was measured by HPLC as previously described herein.

Examples 34-39 represent various blends that lack one or more of the necessary components of the disclosed technology (Reference Examples).

**Table 8**

| Example ID | Blends: API + Excipient(s) | Wt. Ratio of Components | Exposure Conditions | Levetiracetam Acid | Total Impurities |
|---|---|---|---|---|---|
| EX 34 | API | 100 | Initial | ND | 0.01 |
| | | | 12 Day (60C/75% RH) | 0.01 | 0.02 |
| | | | 30 Day (40C/75% RH) | ND | 0.01 |
| EX 35 | API + Carbopol^{®} 974P NF Polymer | 1:0.5 | Initial | 0.02 | 0.03 |
| | | | 12 Day | 2.99 | 3.02 |
| | | | 30 Day | 1.33 | 1.36 |
| EX 36 | API + Carbopol^{®} 971P NF Polymer | 1:0.5 | Initial | 0.01 | 0.03 |
| | | | 12 Day | 1.9 | 1.94 |
| | | | 30 Day | 0.96 | 0.98 |
| EX 37 | API + Carbopol^{®} 71GP NF Polymer | 1:0.5 | Initial | 0.01 | 0.02 |
| | | | 12 Day | 2.83 | 2.86 |
| | | | 30 Day | 1.08 | 1.13 |
| EX 38 | API + Mg(OH)₂ | 1:0.5 | Initial | ND | 0.01 |
| | | | 12 Day | 0.12 | 0.16 |
| | | | 30 Day | 0.04 | 0.05 |
| EX 39 | API + MgO⁻ | 1:0.5 | Initial | ND | 0.01 |
| | | | 12 Day | 0.12 | 0.16 |
| | | | 30 Day | 0.04 | 0.05 |

Blends of Levetiracetam (API) were prepared (at ambient room termperature and RH) in the various blend ratios from the components set forth in Table 9. A sample of each of the blends was subjected to accelerated degradation testing at 40°C and 75% RH for a period of one month. At the end of the storage period, the samples were analyzed for Levetiracetam acid and total impurities using the methodology set forth above.

**Table 9**

| Example ID | Blends: API + Excipient(s) | Wt. Ratio of Components | Exposure Conditions | Levetiracetam Acid | Total Impurities |
|---|---|---|---|---|---|
| EX 40 | API + Carbopol^{®} 971P NF Polymer + Mg(OH)₂⁻ | 1.00:0.22:0.11 | 40°C/75%RH 1 Month | 0.04 | 0.05 |
| EX41 | API + Carbopol^{®} 971P NF Polymer + Mg(OH)₂ | 1.00:0.22:0.15 | 40°C/75%RH 1 Month | 0.03 | 0.04 |
| EX **42** | API + Carbopol^{®} 971P NF Polymer + Mg(OH)₂ | 1.00:0.15:0.15 | 40°C/75%RH 1 Month | 0.03 | 0.04 |
| EX **43** | API + Carbopol^{®} 971P NF Polymer + Mg(OH)₂+ Neusilin^{™} UFL2 | 1.00:0.15:0.15 :0.11 | 40°C/75%RH 1 Month | 0.08 | 0.09 |

**Table 10**

| Example ID | Tablets: API + Excipient(s) | Wt. Ratio of Components | Exposure Conditions | Levetiracetam Acid | Total Impurities |
|---|---|---|---|---|---|
| EX 44 | API + Carbopol^{®} 971P NF Polymer + Mg(OH)₂⁻ | 1.00:0.22:0.11 | 40°C/75%RH 1 Month | 0.02 | 0.03 |
| EX 45 | API + Carbopol^{®} 971P NF Polymer + Mg(OH)₂ | 1.00:0.22:0.15 | 40°C/75%RH 1 Month | 0.01 | 0.02 |
| EX 46 | API + Carbopol^{®} 971P NF Polymer + Mg(OH)₂ | 1.00:0.15:0.15 | 40°C/75%RH 1 Month | 0.01 | 0.02 |
| EX 47 | API + Carbopol^{®} 971P NF Polymer + Mg(OH)₂+ Neusilin^{™} UFL2 | 1.00:0.15:0.15:0.11 | 40°C/75%RH 1 Month | 0.05 | 0.06 |

Blends of Levetiracetam (API) were prepared (at ambient room temperature and RH) in the various blend ratios from the components set forth in Table 10 above. Each blend was tableted and the tablets were subjected to accelerated degradation testing at 40°C and 75% RH for a period of one month. At the end of the storage period, tablet samples were analyzed for Levetiracetam acid and total impurities using the methodology set forth above.

The mention of any document is not an admission that such document qualifies as prior art or constitutes the general knowledge of the skilled person in any jurisdiction. Except in the Examples, or where otherwise explicitly indicated, all numerical quantities in this description specifying amounts of materials, reaction conditions, molecular weights, number of carbon atoms, and the like, are to be understood as modified by the word "about." It is to be understood that the upper and lower amount, range, and ratio limits set forth herein may be independently combined.

As used herein, the transitional term "comprising," which is synonymous with "including," "containing," or "characterized by," is inclusive or open-ended and does not exclude additional, un-recited elements or method steps. However, in each recitation of "comprising" herein, it is intended that the term also encompass, as alternative embodiments, the phrases "consisting essentially of" and "consisting of," where "consisting of" excludes any element or step not specified and "consisting essentially of" permits the inclusion of additional un-recited elements or steps that do not materially affect the basic and novel characteristics of the composition or method under consideration. That is "consisting essentially of" permits the inclusion of substances that do not materially affect the basic characteristics of the composition under consideration.

## Claims

1. A composition comprising:
(a) a drug component comprising a therapeutically effective amount of a drug;
(b) an extended release component comprising a cross-linked polyacrylic acid polymer that is a carbomer homopolymer, carbomer copolymer, carbomer interpolymer, polycarbophil or a mixture thereof; and
(c) a buffering component comprising magnesium hydroxide, magnesium oxide, or any combination thereof;
wherein the drug component makes up at least 50% by weight of the composition.

2. The composition of claim 1 wherein the drug of said drug component is subject to hydrolytic degradation.

3. The composition of any of the claims 1 to 2 wherein the drug of said drug component comprises (i) an ester functional group, (ii) a lactone functional group, (iii) an amide or amide related functional group, (iv) a reactive nitrogen functional group, or (v) any combination thereof.

4. The composition of any of the claims 1 to 3 wherein the drug of said drug component comprises:
(i) Methylphenidate, Aspirin, Procaine, Benzocaine, Physostigmine, Tetracaine, N-methyl-dopa, Scopolamine, Meperidine, Steroid esters such as hydrocortisone sodium succinate, and methylprednisolone sodium succinate, succinylcholine chloride, chlorphenesin carbamate, carmethizole, cyclodisone, Estramustine, Carzelesin, hydrocortisone disodium phosphate, echothiophate Iodide, nitroglycerin, nicorandil, Phosphatidylcholine, phosphatidylethanolamine or any related compound;
(ii) Lovastatin, Simvastatin, Daptomycin, Pilocarpine, Dalvastatin, Warfarin,and camptothecin or any related compound;
(iii) Acetamide, Chloramphenicol, Indomethacin, Lidocaine, Prazosin, Doxazosin, Dibucaine, acetaminophen, lincomycin, sulfacetamide, moricizin, Amoxicillin, Ampicillin, Latamoxef, benzylpenicillin, carbenicillin, phenethicillin, methicillin, cephems, such as cephalothin, cefadroxil, cephradine, and cefotaxime Cefepime, Cefaclor or other penicillins and cephalospoins any combination thereof; peptides, polypeptides and proteins, amide related functional groups in levetiracetam, barbital, phenobarbital, amobarbital, metharbital, allantoin, Obidoxime, Doxorubicin, Tobramycin, or any related compounds;
(iv) Benzodiazepines such as diazepam, oxazepam, nitrazepam, chlordiazepoxide, Triazolam, oxazolam, flutazolam, haloxazolam, cloxazolam, or any combination thereof; biguaninides such as metformin, phenformin, buformin, or any combination thereof; metoprolol, propranolol, bisoprolol, sotalol, atenolol, sulpyrine, furosemide, thiamine hydrochloride,diethylpropion, mitomycin C, zileuton, cifenline, Nitrofurantoin, rifampicin, chlorothiazide, hydrochlorothiazide, 5-azacytidine, cytarabine, or any related compounds;
(v) any combinations thereof.

5. The composition of any of the claims 1 to 4 wherein the drug of said drug component comprises Metformin, Levetiracetam, Metoprolol, or any combination thereof.

6. The composition of any of the claims 1 to 5 wherein the drug component makes up from 50% to 85% by weight of the composition.

7. The composition of any of the claims 1 to 6 wherein:
(a) the drug component makes up 50% to 85% by weight of the drug composition;
(b) the extended release component makes up 3% to 40% by weight of the drug composition;
(c) the buffering component makes up 1% to 20% by weight of the drug composition; and
wherein said composition may optionally further comprise one or more additional additives.

8. The composition of any of the claims 1 to 7 wherein the composition has improved stability, as indicated by a lower level of impurities in the composition after exposure forced degradation testing conditions;
wherein the forced degradation testing conditions comprises exposing the composition, in the form of a tablet, to 60 to 80 degrees C and 75% relative humidity for a period of 5 or 12 days; and
wherein said lower level of impurities is measured by high-pressure liquid chromatography (HPLC) analysis and compared to the level of impurities found when the same composition is tested without said buffering component.

9. The composition of any of the claims 1 to 8 wherein the composition is in the form designed for oral, rectal, nasal, topical, vaginal or parenteral administration or is in a form suitable for administration by inhalation or insufflation.

10. The composition of any of the claims 1 to 8 wherein the composition has a pH level of 4 to 10;
wherein the pH level is measured by making an aqueous dispersion of the tablet using pH-meter; and
wherein the composition exhibits no more than 1% by weight impurities after forced degradation;
wherein forced degradation comprises exposing the composition, in the form of a tablet, to 60 to 80 degrees C and 75% relative humidity for a period of 5 or 12 days; and wherein the level of impurities is measured by highperformance liquid chromatography analysis.

11. The composition of any of the claims 1 to 9 wherein the composition is in the form of a tablet, capsules, granules, beads, or an aqueous dispersion.

12. A method of making a modified release tablet, wherein said method comprises the steps of:
I. combining:
(a) a drug component comprising a therapeutically effective amount of a drug;
(b) an extended release component comprising a cross-linked polyacrylic acid polymer that is a carbomer homopolymer, carbomer copolymer, carbomer interpolymer, polycarbophil or a mixture thereof; and
(c) a buffering component comprising magnesium hydroxide, magnesium oxide, or any combination thereof;
wherein the drug component makes up at least 50% by weight of the composition; and
II. using direct compression, granulation, or a combination thereof, to form the composition into a tablet.

13. The composition of any one of claims 1 to 10 for use as a medicament, wherein said composition has been formed into a tablet using direct compression, granulation, or a combination thereof.

14. The composition for use according to claim 13, wherein the buffering component makes up 1% to 20% by weight of the drug composition.

## Patentansprüche

1. Zusammensetzung, die Folgendes umfasst:
(a) eine Arzneimittelkomponente, die eine therapeutisch wirksame Menge eines Arzneimittels umfasst;
(b) eine Komponente mit verlängerter Freisetzung, die ein vernetztes Polyacrylsäurepolymer umfasst, das ein Carbomerhomopolymer, Carbomer-Copolymer, Carbomermischpolymerisat, Polycarbophil oder eine Mischung davon ist; und
(c) eine Pufferkomponente, die Magnesiumhydroxid, Magnesiumoxid oder eine beliebige Kombination davon umfasst;
wobei die Arzneimittelkomponente wenigstens 50 Gew.-% der Zusammensetzung ausmacht.

2. Zusammensetzung nach Anspruch 1, wobei das Arzneimittel der Arzneimittelkomponente einem hydrolytischen Abbau unterzogen wird.

3. Zusammensetzung nach einem der Ansprüche 1 bis 2, wobei das Arzneimittel der Arzneimittelkomponente (i) eine Ester-funktionelle Gruppe, (ii) eine Lacton-funktionelle Gruppe, (Hi) eine Amid- oder Amid-verwandte funktionelle Gruppe, (iv) eine reaktiver Stickstoff-funktionelle Gruppe (v), oder eine beliebige Kombination davon umfasst.

4. Zusammensetzung nach einem der Ansprüche 1 bis 3, wobei das Arzneimittel der Arzneimittelkomponente Folgendes umfasst:
(i) Methylphenidat, Aspirin, Procain, Benzocain, Physostigmin, Tetracain, N-Methyl-Dopa, Scopolamin, Meperidin, Steroidester wie etwa Hydrocortisonnatriumsuccinat und Methylprednisolonnatriumsuccinat, Succinylcholinchlorid, Chlorphenesincarbamat, Carmethizol, Cyclodison, Estramustin, Carzelesin, Hydrocortisondinatriumphosphat, Echothiophatiodid, Nitroglycerin, Nicorandil, Phosphatidylcholin, Phosphatidylethanolamin oder eine beliebige verwandte Verbindung;
(ii) Lovastatin, Simvastatin, Daptomycin, Pilocarpin, Dalvastatin, Warfarin und Camptothecin oder eine beliebige verwandte Verbindung;
(iii) Acetamid, Chloramphenicol, Indomethacin, Lidocain, Prazosin, Doxazosin, Dibucain, Acetaminophen, Lincomycin, Sulfacetamid, Moricizin, Amoxicillin, Ampicillin, Latamoxef, Benzylpenicillin, Carbenicillin, Phenethicillin, Methicillin, Cepheme wie etwa Cephalothin, Cefadroxil, Cephradin und Cefotaximcefepim, Cefaclor oder andere Penicilline und Cephalosporine, eine beliebige Kombination davon; Peptide, Polypeptide und Proteine, Amid-verwandte funktionelle Gruppen in Levetiracetam, Barbital, Phenobarbital, Amobarbital, Metharbital, Allantoin, Obidoxim, Doxorubicin, Tobramycin oder beliebige verwandte Verbindungen;
(iv) Benzodiazepine wie etwa Diazepam, Oxazepam, Nitrazepam, Chlordiazepoxid, Triazolam, Oxazolam, Flutazolam, Haloxazolam, Cloxazolam oder eine beliebige Kombination davon; Biguanide wie etwa Metformin, Phenformin, Buformin oder eine beliebige Kombination davon; Metoprolol, Propranolol, Bisoprolol, Sotalol, Atenolol, Sulpyrin, Furosemid, Thiaminhydrochlorid, Diethylpropion, Mitomycin C, Zileuton, Cifenlin, Nitrofurantoin, Rifampicin, Chlorothiazid, Hydrochlorothiazid, 5-Azacytidin, Cytarabin oder beliebige verwandte Verbindungen;
(v) eine beliebige Kombination davon.

5. Zusammensetzung nach einem der Ansprüche 1 bis 4, wobei das Arzneimittel der Arzneimittelkomponente Metformin, Levetiracetam, Metoprolol oder eine beliebige Kombination davon umfasst.

6. Zusammensetzung nach einem der Ansprüche 1 bis 5, wobei die Arzneimittelkomponente 50 bis 85 Gew.-% der Zusammensetzung ausmacht.

7. Zusammensetzung nach einem der Ansprüche 1 bis 6, wobei:
(a) die Arzneimittelkomponente 50 bis 85 Gew.-% der Arzneimittelzusammensetzung ausmacht;
(b) die Komponente mit verlängerter Freisetzung 3 bis 40 Gew.-% der Arzneimittelzusammensetzung ausmacht;
(c) die Pufferkomponente 1 bis 20 Gew.-% der Arzneimittelzusammensetzung ausmacht; und
wobei die Zusammensetzung optional ferner einen oder mehrere zusätzliche Zusatzstoffe umfassen kann.

8. Zusammensetzung nach einem der Ansprüche 1 bis 7, wobei die Zusammensetzung eine verbesserte Stabilität aufweist, wie durch einen geringeren Wert von Verunreinigungen in der Zusammensetzung nach einer Aussetzung von Testbedingungen eines erzwungenen Abbaus angezeigt wird;
wobei die Testbedingungen des erzwungenen Abbaus ein Aussetzen der Zusammensetzung in der Ausbildung einer Tablette 60 bis 80 Grad C und 75 % relativer Feuchtigkeit für einen Zeitraum von 5 oder 12 Tagen umfassen; und
wobei der niedrigere Wert von Verunreinigungen durch Hochdruckflüssigkeitschromatographie (high-pressure liquid chromatography - HPLC)-Analyse gemessen und mit dem Wert von Verunreinigungen verglichen wird, der gefunden wird, wenn die gleiche Zusammensetzung ohne die Pufferkomponente getestet wird.

9. Zusammensetzung nach einem der Ansprüche 1 bis 8, wobei die Zusammensetzung in der Ausbildung vorliegt, die für eine orale, rektale, nasale, topische, vaginale oder parenterale Verabreichung bestimmt ist, oder in einer Ausbildung vorliegt, die für die Verabreichung durch eine Inhalation oder Insufflation geeignet ist.

10. Zusammensetzung nach einem der Ansprüche 1 bis 8, wobei die Zusammensetzung einen pH-Wert von 4 bis 10 aufweist;
wobei der pH-Wert durch Herstellen einer wässrigen Dispersion der Tablette unter Verwendung eines pH-Meters gemessen wird; und
wobei die Zusammensetzung nach dem erzwungenen Abbau nicht mehr als 1 Gew.-% Verunreinigungen aufweist;
wobei der erzwungene Abbau das Aussetzen der Zusammensetzung in der Ausbildung einer Tablette 60 bis 80 Grad C und 75 % relativer Feuchtigkeit für einen Zeitraum von 5 oder 12 Tagen umfasst; und wobei der Wert von Verunreinigungen durch Hochleistungsflüssigkeitschromatographieanalyse gemessen wird.

11. Zusammensetzung nach einem der Ansprüche 1 bis 9, wobei die Zusammensetzung in der Ausbildung einer Tablette, Kapseln, Körner, Perlen oder einer wässrigen Dispersion vorliegt.

12. Verfahren zum Herstellen einer Tablette mit modifizierter Freisetzung, wobei das Verfahren die folgenden Schritte umfasst:
I. kombinieren:
(a) einer Arzneimittelkomponente, die eine therapeutisch wirksame Menge eines Arzneimittels umfasst;
(b) einer Komponente mit verlängerter Freisetzung, die ein vernetztes Polyacrylsäurepolymer umfasst, das ein Carbomerhomopolymer, Carbomer-Copolymer, Carbomermischpolymerisat, Polycarbophil oder eine Mischung davon ist; und
(c) einer Pufferkomponente, die Magnesiumhydroxid, Magnesiumoxid oder eine beliebige Kombination davon umfasst;
wobei die Arzneimittelkomponente wenigstens 50 Gew.-% der Zusammensetzung ausmacht; und
II. unter Verwendung von direkter Kompression, Granulierung oder einer Kombination davon, um die Zusammensetzung zu einer Tablette auszubilden.

13. Zusammensetzung nach einem der Ansprüche 1 bis 10 zur Verwendung als ein Arzneimittel, wobei die Zusammensetzung unter Verwendung von direkter Kompression, Granulierung oder einer Kombination davon zu einer Tablette ausgebildet worden ist.

14. Zusammensetzung zur Verwendung nach Anspruch 13, wobei die Pufferkomponente 1 bis 20 Gew.-% der Arzneimittelzusammensetzung ausmacht.

## Revendications

1. Composition comprenant :
(a) un composant médicamenteux comprenant une quantité thérapeutiquement efficace d'un médicament ;
(b) un composant à libération prolongée comprenant un polymère d'acide polyacrylique réticulé qui est un homopolymère carbomère, un copolymère carbomère, un interpolymère carbomère, un polycarbophile ou un mélange de ceux-ci ; et
(c) un composant tampon comprenant de l'hydroxyde de magnésium, de l'oxyde de magnésium ou toute combinaison de ceux-ci ;
le composant médicamenteux constituant au moins 50 % en poids de la composition.

2. Composition selon la revendication 1, dans laquelle le médicament dudit composant médicamenteux est soumis à une dégradation hydrolytique.

3. Composition selon l'une quelconque des revendications 1 à 2, dans laquelle le médicament dudit composant médicamenteux comprend (i) un groupe fonctionnel ester, (ii) un groupe fonctionnel lactone, (Hi) un groupe fonctionnel amide ou apparenté à un amide, (iv) un réactif un groupe fonctionnel azoté, ou (v) toute combinaison de ceux-ci.

4. Composition selon l'une quelconque des revendications 1 à 3, dans laquelle le médicament dudit composant médicamenteux comprend :
(i) du méthylphénidate, de l'aspirine, de la procaïne, de la benzocaïne, de la physostigmine, de la tétracaïne, du N-méthyl-dopa, de la scopolamine, de la mépéridine, des esters de stéroïdes tels que du succinate sodique d'hydrocortisone et du succinate sodique de méthylprednisolone, du chlorure de succinylcholine, du carbamate de chlorphénésine, du carméthizole, de la cyclodisone, de l'estramustine, de la carzélésine, du phosphate disodique d'hydrocortisone, de l'iodure d'échothiophate, de la nitroglycérine, du nicorandil, de la phosphatidylcholine, de la phosphatidyléthanolamine ou tout composé apparenté ;
(ii) de la lovastatine, de la simvastatine, de la daptomycine, de la pilocarpine, de la dalvastatine, de la warfarine et de la camptothécine ou tout composé apparenté ;
(iii) de l'acétamide, du chloramphénicol, de l'indométhacine, de la lidocaïne, de la prazosine, de la doxazosine, de la dibucaïne, de l'acétaminophène, de la lincomycine, de la sulfacétamide, de la moricizine, de l'amoxicilline, de l'ampicilline, du latamoxef, de la benzylpénicilline, de la carbénicilline, de la phénéticilline, de la méthicilline, des céphèmes, tels que de la céphalothine, du céfadroxil, de la céphradine et de la céfotaxime, de la céfépime, du céfaclor ou autres pénicillines et des céphalospores, toute combinaison de ceux-ci ; des peptides, des polypeptides et des protéines, des groupes fonctionnels apparentés aux amides dans le lévétiracétam, le barbital, le phénobarbital, l'amobarbital, le métharbital, l'allantoïne, l'obidoxime, la doxorubicine, la tobramycine ou tout composé apparenté ;
(iv) des benzodiazépines telles que le diazépam, l'oxazépam, le nitrazépam, le chlordiazépoxide, le triazolam, l'oxazolam, le flutazolam, l'haloxazolam, le cloxazolam, ou toute combinaison de ceux-ci ; les biguaninides tels que la metformine, la phenformine, la buformine ou toute combinaison de celles-ci ; le métoprolol, le propranolol, le bisoprolol, le sotalol, l'aténolol, le sulpyrine, le furosémide, le chlorhydrate de thiamine, le diéthylpropion, la mitomycine C, le zileuton, la cifenline, la nitrofurantoïne, la rifampicine, la chlorothiazide, l'hydrochlorothiazide, la 5-azacytidine, la cytarabine ou tout composé apparenté ;
(v) toute combinaison de ceux-ci.

5. Composition selon l'une quelconque des revendications 1 à 4, dans laquelle le médicament dudit composant médicamenteux comprend de la metformine, du lévétiracétam, du métoprolol ou toute combinaison de ceux-ci.

6. Composition selon l'une quelconque des revendications 1 à 5, dans laquelle le composant médicamenteux constitue de 50 % à 85 % en poids de la composition.

7. Composition selon l'une quelconque des revendications 1 à 6, dans laquelle :
(a) le composant médicamenteux représente 50 % à 85 % en poids de la composition médicamenteuse ;
(b) le composant à libération prolongée représente 3 % à 40 % en poids de la composition médicamenteuse ;
(c) le composant tampon représente 1 % à 20 % en poids de la composition médicamenteuse ; et
ladite composition pouvant éventuellement comprendre en outre un ou plusieurs additifs supplémentaires.

8. Composition selon l'une quelconque des revendications 1 à 7, dans laquelle la composition présente une stabilité améliorée, comme indiqué par un niveau inférieur d'impuretés dans la composition après exposition aux conditions de test de dégradation forcée ;
les conditions de test de dégradation forcée comprenant l'exposition de la composition, sous la forme d'un comprimé, à 60 à 80 degrés C et 75 % d'humidité relative pendant une période de 5 ou 12 jours ; et
ledit niveau inférieur d'impuretés étant mesuré par analyse par chromatographie liquide à haute pression (HPLC) et comparé au niveau d'impuretés trouvé lorsque la même composition est testée sans ledit composant tampon.

9. Composition selon l'une quelconque des revendications 1 à 8, dans laquelle la composition est sous la forme conçue pour l'administration orale, rectale, nasale, topique, vaginale ou parentérale ou est sous une forme appropriée pour l'administration par inhalation ou insufflation.

10. Composition selon l'une quelconque des revendications 1 à 8, dans laquelle la composition a un niveau de pH de 4 à 10 ;
le niveau de pH étant mesuré en réalisant une dispersion aqueuse du comprimé à l'aide d'un pH-mètre ; et
la composition ne présentant pas plus de 1 % en poids d'impuretés après dégradation forcée ;
la dégradation forcée comprenant l'exposition de la composition, sous la forme d'un comprimé, à 60 à 80 degrés C et 75 % d'humidité relative pendant une période de 5 ou 12 jours ; et le niveau d'impuretés étant mesuré par analyse par chromatographie liquide à haute performance.

11. Composition selon l'une quelconque des revendications 1 à 9, dans laquelle la composition est sous la forme d'un comprimé, de capsules, de granulés, de perles ou d'une dispersion aqueuse.

12. Procédé de fabrication d'un comprimé à libération modifiée, ledit procédé comprenant les étapes consistant à :
I. combiner :
(a) un composant médicamenteux comprenant une quantité thérapeutiquement efficace d'un médicament ;
(b) un composant à libération prolongée comprenant un polymère d'acide polyacrylique réticulé qui est un homopolymère carbomère, un copolymère carbomère, un interpolymère carbomère, un polycarbophile ou un mélange de ceux-ci ; et
(c) un composant tampon comprenant de l'hydroxyde de magnésium, de l'oxyde de magnésium ou toute combinaison de ceux-ci ;
le composant médicamenteux constituant au moins 50 % en poids de la composition ; et
II. utiliser une compression directe, une granulation ou une combinaison de celles-ci, pour transformer la composition en un comprimé.

13. Composition selon l'une quelconque des revendications 1 à 10 destinée à être utilisée comme médicament, dans laquelle ladite composition a été formée en un comprimé à l'aide d'une compression directe, d'une granulation ou d'une combinaison de celles-ci.

14. Composition à utiliser selon la revendication 13, dans laquelle le composant tampon représente 1 % à 20 % en poids de la composition médicamenteuse.
